# Europäisches Patentamt

# European Patent Office

# Office européen des brevets

(19)

(11) Veröffentlichungsnummer: **0 073 922**
**B1**

(12) **EUROPÄISCHE PATENTSCHRIFT**

(45) Veröffentlichungstag der Patentschrift:
04.12.85

(21) Anmeldenummer: 82106850.9

(22) Anmeldetag: 29.07.82

(51) Int. Cl.⁴: **B 01 J 31/40, C 07 C 51/54**

(54) **Verfahren zur Reinigung und Rückgewinnung der bei der Carbonylierung von Methylacetat und/oder Dimethylether anfallenden, verunreinigten Katalysatorlösung.**

(30) Priorität: 31.08.81 DE 3134347

(43) Veröffentlichungstag der Anmeldung:
16.03.83 Patentblatt 83/11

(45) Bekanntmachung des Hinweises auf die Patenterteilung:
04.12.85 Patentblatt 85/49

(84) Benannte Vertragsstaaten:
AT BE CH DE FR GB IT LI NL

(56) Entgegenhaltungen:
EP - A - 0 026 280
DE - C - 869 203
US - A - 3 065 242
US - A - 3 634 291

(73) Patentinhaber: HOECHST AKTIENGESELLSCHAFT,
Postfach 80 03 20, D-6230 Frankfurt am Main 80 (DE)

(72) Erfinder: Erpenbach, Heinz, Dr., Oberbuschweg 22,
D-5000 Köln 50 (DE)
Erfinder: Gehrmann, Klaus, Dr.,
Geschwister-Scholl-Strasse 32, D-5042 Erftstadt (DE)
Erfinder: Lork, Winfried,
Siegfried-von-Westerburg-Strasse 14, D-5042 Erftstadt
(DE)
Erfinder: Prinz, Peter, von Geyr-Ring 104, D-5030 Hürth
(DE)

BUNDESDRUCKEREI BERLIN

0 073 922

**Beschreibung**

Die Erfindung betrifft ein Verfahren zur Reinigung und Rückgewinnung der bei der Carbonylierung von Methylacetat und/oder Dimethylether anfallenden, verunreinigten Katalysatorlösung, welche Carbonylkomplexe von Edelmetallen der Gruppe VIII des Periodensystems der Elemente, quaternäre heterocyclische aromatische Stickstoffverbindungen oder quaternäre Organophosphorverbindungen als organische Promotoren, ggf. Verbindungen carbonylbildender Nicht-Edelmetalle als anorganische Promotoren, undestillierbare organische Verunreinigungen sowie Essigsäure, Essigsäureanhydrid und Ethylidendiacetat enthält.

Die Rückgewinnung von Rhodium- bzw. Rhodiumcarbonylkomplexen aus mit Rückständen verunreinigten Katalysatorsystemen oder Destillationssümpfen ist im wesentlichen bei Hydroformylierungsverfahren bekannt. So wird in der DE-C-1 290 535 der rhodiumhaltige Sumpf der Hydroformylierung bei erhöhter Temperatur mit wäßrigen organischen Säuren behandelt, wobei der Rhodiumkomplex in Lösung geht und so von der organischen Phase abgetrennt wird. Dabei wird eine Rhodiumausbeute von 82 bis 94% erzielt.

In der DE-B-1 295 537 wird der rhodiumhaltige Reaktionssumpf mit Wasserdampf von 2—31 bar und 100—250°C behandelt. Durch diese Maßnahme wird der Katalysator zersetzt und der anfallende Rhodiumschlamm, der als solcher für den Carbonylierungsprozeß nicht wieder einsetzbar ist, mittels Druckfiltration gewonnen.

Andere Möglichkeiten der Rhodiumwiedergewinnung bzw. der Katalysatorregenerierung beschreiben die DE-A-2 448 005 und 2 614 799. Hierbei werden die verunreinigten Destillationssümpfe aus der Hydroformylierung zur Zerstörung der Rhodium- bzw. Iridiumcarbonylkomplexe und Abbau der Rückstände zunächst einer Behandlung mit sauerstoffhaltigen Mineralsäuren und Peroxiden unterzogen. Nach Zersetzen der überschüssigen Peroxide werden die wäßrigen Metallsalzlösungen unterschiedlich aufgearbeitet. Gemäß DE-A-2 448 005 werden die Metalle nach Zusatz eines wasserlöslichen organischen Lösemittels, einer Halogenwasserstoffsäure oder eines Alkalihalogenids sowie eines tertiären Phosphins zur wäßrigen Rhodium- bzw. Iridiumsalzlösung bei 0—150°C und 1—250 bar mit Kohlenmonoxid oder auch zusätzlich unter hydrierenden Bedingungen als Rhodium- bzw. Iridiumcarbonylkomplexe aus der Lösung abgeschieden. Gemäß DE-A-2 614 799 wird aus der wäßrigen Lösung das Rhodium$^{+++}$ an einem Kationenaustauscher absorbiert, danach mit Salzsäure desorbiert. Aus der salzsauren Lösung wird nach Zusatz von tertiären Phosphinen und Behandeln mit CO und ggf. Wasserstoff das Rhodium als Carbonylkomplex ausgefällt.

Für die Reinigung und Rückgewinnung der Edelmetallcarbonylkomplexe (Rh, Ir, Pd, Ru) und ihrer Promotoren aus der bei der Carbonylierung von Methylacetat oder Dimethylether (z. B. gemäß den Verfahren der DE-A-2 450 965, 2 836 084, 2 939 839 oder 2 941 232) anfallenden verunreinigten Katalysatorlösung sind die beschriebenen Methoden nicht anwendbar. Die für die genannte Carbonylierungsreaktion verwendete Katalysatorlösung besteht aus 0,1—10 Gew.-% Edelmetallcarbonylkomplex, 40—70 Gew.-% organischen Promotoren, 1—10 Gew.-% undestillierbaren organischen Verunreinigungen und 20—40 Gew.-% Essigsäure, Essigsäureanhydrid und Ethylidendiacetat. Diese Zusammensetzung macht deutlich, daß die Katalysatorlösung bis zu 80 Gew.-% undestillierbare Substanzen enthalten kann. Ein oxidativer Abbau des Edelmetallcarbonylkomplexes nach Destillation der flüchtigen Bestandteile würde gleichzeitig die Zerstörung des gesamten organischen Promotoranteils bedeuten, wodurch der Nutzen einer Katalysatoraufarbeitung von vorneherein in Frage gestellt wäre.

Auch eine Behandlung des Rückstandes mit wäßrigen organischen Säuren ist von Nachteil, da hierbei neben dem Edelmetallcarbonylkomplex auch der größte Teil der undestillierbaren organischen Verunreinigungen in Lösung geht und nicht vom Katalysator und Promotor abgetrennt werden kann.

Die Zersetzung des Katalysators mit Wasserdampf bei höherer Temperatur führt zu Edelmetall in elementarer Form, das nur unter hohem Aufwand wieder in den aktiven Katalysatorkomplex zurückgeführt werden kann. Darüber hinaus fällt aber gleichzeitig auch der in Wasser unlösliche organische Rückstand an, der vom elementaren Edelmetall nicht ohne weiteres abzutrennen ist.

Die vorliegende Erfindung umgeht die aufgezeigten Nachteile und beschreibt ein Verfahren, das es mit Hilfe destillativer und extraktiver Methoden ermöglicht, die bei der Carbonylierung von Methylacetat und/oder Dimethylether eingesetzte und im Laufe des Prozesses verunreinigte Katalysatorlösung so aufzuarbeiten, daß unter Ausschleusung der undestillierbaren organischen Verunreinigungen sowohl der Edelmetall-Carbonyl-Komplex als auch seine Promotoren dem Katalysatorkreislauf wieder zugeführt werden können. Daneben wird im Vergleich zu den bekannten Aufarbeitungsverfahren durch den Kreislauf der zur Aufarbeitung eingesetzten Extraktionsmittel eine größere Umweltbelastung durch Abfallstoffe vermieden. Lediglich die im Prozeß und durch den Reaktionsablauf gebildeten undestillierbaren organischen Verunreinigungen werden ausgeschleust und müssen entsprechend dem Stand der Technik, z. B. durch Verbrennung, beseitigt werden.

Im einzelnen ist die Erfindung nunmehr dadurch gekennzeichnet, daß man die flüchtigen Bestandteile aus der Katalysatorlösung abdestilliert, den verbleibenden festen Destillationsrückstand durch Extraktion mit aliphatischen Ethern von den organischen Verunreinigungen befreit und das ungelöst zurückbleibende Gemisch aus Edelmetall-Carbonyl-Komplex, organischen und ggf. anorganischen Promotoren abfiltriert, trocknet und in die Carbonylierungsreaktion zurückführt.

2

Das Verfahren der Erfindung kann weiterhin bevorzugt und wahlweise dadurch gekennzeichnet sein, daß man

a) die Behandlung des Destillationsrückstands mit Ether bei 30−90° C vornimmt;
b) je Gewichtsteil Destillationsrückstand 50 bis 200 Gewichtsteile Ether einsetzt;
c) den festen Destillationsrückstand mit Diethyl- oder Diisopropylether extrahiert.

Zur Herkunft der verunreinigten Katalysatorlösung ist zu sagen, daß die aus einem Carbonylierungsreaktor abfließende Reaktionsmischung destillativ in die gewünschten Endprodukte, besonders Essigsäureanhydrid, Essigsäure und/oder Ethylidendiacetat, und nichtumgesetzte, im Kreislauf geführte Ausgangsstoffe einerseits sowie die als Sumpfprodukt anfallende Katalysatorlösung andererseits aufgetrennt wird. Ein Teilstrom dieser mit der Zeit verunreinigten Katalysatorlösung wird aus dem Kreislauf der Katalysatorlösung entnommen und erfindungsgemäß destillativ, vorzugsweise bei 100−120° C und 1−100 mbar, von den flüchtigen Bestandteilen wie Essigsäure, Essigsäureanhydrid und Ethylidendiacetat befreit.

Die verunreinigten Katalysatorlösungen enthalten als Edelmetalle im allgemeinen Rhodium, Iridium, Palladium und/oder Ruthenium, die als Carbonylkomplexe wie z. B.

$$[CH_3P(C_4H_9)_3]_2Rh(CO)I_5 \quad oder \quad CH_3P(C_4H_9)_3Rh(CO)_2I_2$$

vorliegen. Die Katalysatorlösungen enthalten ferner als organische Promotoren bevorzugt einen oder mehrere der folgenden heterocyclischen aromatischen Stickstoffverbindungen oder Organophosphorverbindungen:

1. N-Methylpyridiniumjodid, N,N-Dimethylimidazoliumjodid, N-Methyl-3-picoliniumjodid, N-Methyl-2,4-lutidiniumjodid, N-Methyl-3,4-lutidiniumjodid, N-Methyl-chinoliniumjodid;
2. Tri-n-butyl-methyl-phosphoniumjodid, Trioctyl-methyl-phosphoniumjodid, Trilauryl-methyl-phosphoniumjodid, Triphenyl-methyl-phosphoniumjodid.

Schließlich kann die Katalysatorlösung als anorganische Promotoren Verbindungen der carbonylbildenden Nicht-Edelmetalle Ce, Ti, Zr, Hf, Ge, Sn, Pb, V, Nb, Ta, As, Sb, Bi, Cr, Mo, W, Mn, Re, Fe, Co, Ni enthalten.

Der verbleibende Destillationsrückstand wird sodann mit einem aliphatischen Ether bei 30−90° C extrahiert. Hierbei werden die bei der Reaktion gebildeten undestillierbaren organischen Verunreinigungen aus dem Destillationsrückstand in einem einzigen Verfahrensschritt herausgelöst, während der Edelmetall-Carbonylkomplex mit dem oder den Promotoren ungelöst als Rückstand verbleibt. Edelmetall-Carbonyl-Komplex und Promotoren können nach Entfernen des noch anhaftenden Ethers wieder in die Carbonylierung eingeführt werden. Die in der Etherphase gelösten undestillierbaren organischen Verunreinigungen werden nach Abtreiben des Ethers in einer Verbrennungsanlage vernichtet, während der Ether zur erneuten Extraktion wieder verwendet werden kann. Das Verfahren der Erfindung kann sowohl in kontinuierlicher als auch diskontinuierlicher Betriebsweise durchgeführt werden.

## Beispiel 1

Dem aus Rhodiumcarbonylkomplex (L₂Rh(CO)I₅; L = Ligand), Tri-n-butylmethylphosphoniumjodid als organischem Promotor, Essigsäureanhydrid, Essigsäure sowie Ethylidendiacetat und organischen Verunreinigungen bestehenden Katalysatorkreislauf der Methylacetatcarbonylierung werden 250 g Katalysatorlösung entnommen und unter vermindertem Druck von etwa 2 mbar sowie einer Blasentemperatur bis zu 120° C von den destillierbaren Anteilen befreit. Dabei werden 81,5 g (32,6 Gew.-%) Destillat (25,8 Gew.-% Essigsäure, 73,8 Gew.-% Essigsäureanhydrid und 0,4 Gew.-% Ethylidendiacetat) und 168,5 g Destillationsrückstand mit 0,433 g Rhodium und 145,71 g Tri-n-butylmethylphosphoniumjodid (= 2 Gew.-% Rh-carbonylkomplex und 58,3 Gew.-% TBMPJ, ber. auf 250 g Katalysatorlösung) erhalten. Der Destillationsrückstand wird zermörsert und zur Entfernung der undestillierbaren organischen Verunreinigungen im Soxhlet-Apparat mit 500 ml Diisopropylether bei Siedetemperatur des Ethers extrahiert. Nach drei- bis fünfstündiger Extraktion und anschließender Trocknung wird der im Ether unlösliche Rückstand mit 152 g ermittelt. Sein Rhodiumgehalt beträgt 0,430 g. Der etherunlösliche Rückstand enthält damit nahezu den gesamten in den Reinigungsprozeß eingesetzten Rhodiumcarbonylkomplex sowie den organischen Promotor und kann als solcher wieder in die Reaktion eingesetzt werden. Die Diisopropyletherphase wird eingedampft, wobei 16 g undestillierbare organische Verunreinigungen (= 6,4 Gew.-%, ber. auf 250 g Katalysatorlösung) mit einem Rhodiumgehalt von 0,012 Gew.-% und frei von organischem Promotor im Kolben zurückbleiben, woraus sich für den Reinigungsprozeß eine Rhodiumausbeute von 99,5% errechnet. Der abdestillierte Ether wird zur erneuten Reinigung wieder verwendet.

3

## Beispiel 2

Dem aus Essigsäureanhydrid, Essigsäure, Ethylidendiacetat, Rhodiumcarbonylkomplex (LRh(CO)$_2$I$_2$; L = Ligand), Tri-n-butylmethylphosphoniumjodid und undestillierbaren organischen Verunreinigungen bestehenden Katalysatorkreislauf der Dimethylethercarbonylierung werden 250 g Katalysatorlösung entnommen. Unter vermindertem Druck von 2 mbar und einer Blasentemperatur bis zu 120° C werden die destillierbaren Anteile abgetrennt. Dabei werden von der eingesetzten Katalysatorlösung 72 g (28,8 Gew.-%) Destillat (31 Gew.-% Essigsäure, 68,6 Gew.-% Essigsäureanhydrid und 0,4 Gew.-% Ethylidendiacetat) und 178 g Destillationsrückstand mit 1,718 g Rhodium und 156,3 g Tri-n-butylmethylphosphoniumjodid (= 4,35 Gew.-% Rh-carbonyl-Komplex und 62,5 Gew.-% TBMPJ, ber. auf 250 g Katalysatorlösung) erhalten. Der Destillationsrückstand wird vermahlen und zur Entfernung von undestillierbaren organischen Verunreinigungen im Soxhlet-Apparat mit 500 ml Diethylether beim Siedepunkt des Ethers extrahiert. Nach drei- bis fünfstündiger Extraktion und anschließender Trocknung wird ein etherunlöslicher Rückstand von 167,5 g erhalten. Der Rhodiumgehalt beträgt 1,70 g, entsprechend einer Ausbeute von 99,0%. Der etherunlösliche Rückstand enthält nahezu den gesamten in den Reinigungsprozeß eingesetzten Rhodiumcarbonylkomplex sowie den organischen Promotor und wird in die Reaktion zurückgeführt.

Die Diethyletherphase wird eingedampft, wobei 10,29 g undestillierbare organische Verunreinigungen (= 4,12 Gew.-%, ber. auf 250 g Katalysatorlösung) im Kolben verbleiben. Laut Analyse besitzt dieser Rückstand noch einen Rhodiumgehalt von 0,09 Gew.-%. Der abdestillierte Diethylether kann für nachfolgende Versuche Wiederverwendung finden.

## Beispiel 3

Dem aus Rhodiumcarbonylkomplex (L$_2$Rh(CO)I$_5$; L = Ligand), N,N-Dimethylimidazoliumjodid, undestillierbaren organischen Verunreinigungen, Essigsäure, Essigsäureanhydrid und Ethylidendiacetat bestehenden Katalysatorkreislauf der Methylacetatcarbonylierung werden 250 g Katalysatorlösung entnommen. Unter vermindertem Druck von 2 mbar und einer Blasentemperatur bis zu 120° C werden die destillierbaren Anteile entfernt. Dabei werden von der eingesetzten Katalysatorlösung 64 g (25,6 Gew.-%) Destillat (29,3 Gew.-% Essigsäure, 70,3 Gew.-% Essigsäureanhydrid und 0,4 Gew.-% Ethylidendiacetat) sowie 186 g Destillationsrückstand mit 0,525 g Rhodium und 161 g N,N-Dimethylimidazoliumjodid (= 1,96 Gew.-% Rh-carbonyl-Komplex und 64,4 Gew.-% DMIJ, ber. auf 250 g Katalysatorlösung) erhalten. Der Destillationsrückstand wird zerkleinert und zur Entfernung von undestillierbaren organischen Verunreinigungen im Soxhlet-Apparat mit 500 ml Diethylether bei der Siedetemperatur des Ethers extrahiert. Nach drei- bis fünfstündiger Extraktion und anschließender Trocknung wird ein etherunlöslicher Rückstand von 167 g erhalten. Der Rhodiumgehalt beträgt 0,520 g, entsprechend einer Ausbeute von 99%. Der etherunlösliche Rückstand enthält nahezu den gesamten in den Reinigungsprozeß eingesetzten Rhodiumcarbonylkomplex sowie den organischen Promotor und wird wieder in die Reaktion zurückgeführt.

Die Diethyletherphase wird eingedampft, wobei 19 g undestillierbare organische Verunreinigungen (= 7,6 Gew.-%, ber. auf 250 g Katalysatorlösung) im Kolben verbleiben. Laut Analyse besitzt dieser Rückstand noch einen Rhodiumgehalt von 0,026 Gew.-%. Die abdestillierte Etherphase wird zur erneuten Extraktion benutzt.

## Beispiel 4

Dem aus Palladiumcarbonylkomplex, Tri-n-butylmethylphosphoniumjodid als organischem Promotor, undestillierbaren organischen Verunreinigungen, Essigsäure, Essigsäureanhydrid und Ethylidendiacetat bestehenden Katalysatorkreislauf der Methylacetatcarbonylierung werden 250 g Katalysatorlösung entnommen und unter vermindertem Druck von 2 mbar sowie einer Blasentemperatur bis zu 120° C von den destillierbaren Anteilen befreit. Dabei werden 80 g (32 Gew.-%) Destillat (51,9 Gew.-% Essigsäure, 10 Gew.-% Essigsäureanhydrid und 38,1 Gew.-% Ethylidendiacetat) und 170 g Destillationsrückstand mit 0,85 g Palladium und 152 g Tri-n-butylmethylphosphoniumjodid (= 2 Gew.-% Pdcarbonylkomplex und 60,8 Gew.-% TBMPJ, ber. auf 250 g Katalysatorlösung) erhalten. Der Destillationsrückstand wird zerkleinert und zur Entfernung der undestillierbaren organischen Verunreinigungen im Soxhlet-Apparat mit 500 ml Diisopropylether bei Siedetemperatur des Ethers extrahiert. Nach 4stündiger Extraktion und anschließender Trocknung wird ein etherunlöslicher Rückstand von 159 g erhalten. Sein Palladiumgehalt beträgt 0,84 g entsprechend einer Ausbeute von 98,8%. Der etherunlösliche Rückstand enthält nahezu den gesamten in den Reinigungsprozeß eingesetzten Palladiumcarbonylkomplex sowie den organischen Promotor und wird wieder in die Reaktion eingesetzt.

Die Diisopropyletherphase wird eingedampft, wobei 11 g undestillierbare organische Verunreinigungen (= 4,4 Gew.-%, ber. auf 250 g Katalysatorlösung) mit einem Pd-Gehalt von 0,03 Gew.-% im Kolben verbleiben. Der abdestillierte Ether wird zur erneuten Extraktion benutzt.

# 0 073 922

## Patentansprüche

1. Verfahren zur Reinigung und Rückgewinnung der bei der Carbonylierung von Methylacetat und/oder Dimethylether anfallenden verunreinigten Katalysatorlösung, welche Carbonylkomplexe von Edelmetallen der Gruppe VIII des Periodensystems der Elemente, quaternäre heterocyclische aromatische Stickstoffverbindungen oder quaternäre Organophosphorverbindungen als organische Promotoren, ggf. Verbindungen carbonylbildender Nicht-Edelmetalle als anorganische Promotoren, undestillierbare organische Verunreinigungen sowie Essigsäure, Essigsäureanhydrid und Ethylidendiacetat enthält, dadurch gekennzeichnet, daß man die flüchtigen Bestandteile aus der Katalysatorlösung abdestilliert, den verbleibenden festen Destillationsrückstand durch Extraktion mit aliphatischen Ethern von den organischen Verunreinigungen befreit und das ungelöst zurückbleibende Gemisch aus Edelmetall-Carbonyl-Komplex, organischen und ggf. anorganischen Promotoren abfiltriert, trocknet und in die Carbonylierungsreaktion zurückführt.

2. Verfahren nach Anspruch 1, dadurch gekennzeichnet, daß man die Behandlung des Destillationsrückstands mit Ether bei 30—90° C vornimmt.

3. Verfahren nach einem der vorhergehenden Ansprüche, dadurch gekennzeichnet, daß man je Gewichtsteil Destillationsrückstand 50 bis 200 Gewichtsteile Ether einsetzt.

4. Verfahren nach einem der vorhergehenden Ansprüche, dadurch gekennzeichnet, daß man den festen Destillationsrückstand mit Diethyl- oder Diisopropylether extrahiert.


## Claims

1. Process for purifying and recovering the contaminated catalyst solution which is obtained in the carbonylation of methyl acetate and/or dimethylether, containing carbonyl complexes of noble metals of group VIII of the Periodic System of the elements, quaternary heterocyclic aromatic nitrogen compounds or quaternary organophosphorus compounds as organic promoters, and optionally compounds of carbonylyielding non-noble metals as inorganic promoters, undistillable organic contaminants as well as acetic acid, acetic anhydride and ethylidene diacetate, which comprises: distillatively freeing the catalyst solution from its volatile constituents and freeing the remaining solid distillation residue from the organic contaminants by extracting it with aliphatic ethers; filtering off the remaining undissolved mixture of noble metal/carbonyl-complex, organic promoters and optionally inorganic promoters, drying and recycling it into the carbonylation reaction.

2. Process as claimed in claim 1, wherein the treatment of the distillation residue with ether is effected at 30 to 90° C.

3. Process as claimed in any of the preceding claims, wherein 50 to 200 parts by weight ether are used per part by weight distillation residue.

4. Process as claimed in any of the preceding claims, wherein the solid distillation residue is extracted with diethyl- or diisopropylether.


## Revendications

1. Procédé de purification et de récupération de la solution catalytique contaminée obtenue dans la carbonylation d'acétate de méthyle et/ou d'éther diméthylique et contenant des complexes carbonyles de métaux nobles appartenant au groupe VIII de la Classification Périodique des éléments, des composés azotés aromatiques hétérocycliques quaternaires ou des composés organophosphorés quaternaires comme activeurs organiques et, éventuellement, des composés de métaux non nobles formant des carbonyles comme activeurs inorganiques, des impuretés organiques non distillables ainsi que de l'acide acétique, de l'anhydride acétique et du diacétate d'éthylidène, caractérisé en ce que l'on sépare par distillation les constituants volatils de la solution catalytique, on débarrasse le résidu de distillation solide restant des impuretés organiques par extraction par des éthers aliphatiques, on sépare par filtration le mélange indissous constitué par le complexe métal noble-carbonyle et par les activeurs organiques et, éventuellement, inorganiques, on le sèche et on le renvoie dans la réaction de carbonylation.

2. Procédé selon la revendication 1, caractérisé en ce que l'on effectue le traitement par l'éther du résidu de distillation à 30—90° C.

3. Procédé selon l'une des revendications précédentes, caractérisé en ce que l'on utilise 50—200 parties en poids d'éther par partie en poids de résidu de distillation.

4. Procédé selon l'une des revendications précédentes, caractérisé en ce que l'on extrait le résidu de distillation solide par l'éther diéthylique ou diisopropylique.